# EUROPÄISCHE PATENTSCHRIFT

(11) **EP 2 791 155 B1**
(45) Veröffentlichungstag und Bekanntmachung des Hinweises auf die Patenterteilung: **17.08.2016**
(21) Anmeldenummer: 12808315.1
(22) Anmeldetag: 12.12.2012
(51) Int. Cl.: C07C 235/80, C07F 15/02, C08G 18/76, C08G 18/22

(54) **EISEN(III)-KOMPLEXVERBINDUNGEN ALS KATALYSATOREN FÜR POLYURETHAN-ZUSAMMENSETZUNGEN**
IRON(III) COMPLEXES AS CATALYSTS FOR POLYURETHANE COMPOSITIONS
COMPOSÉS À BASE DE COMPLEXES DE FER (III) COMME CATALYSEURS DE COMPOSITIONS DE POLYURÉTHANE

(30) Priorität: 12.12.2011 EP 11193062
(43) Veröffentlichungstag der Anmeldung: 22.10.2014
(73) Patentinhaber: Sika Technology AG, 6340 Baar (CH)
(72) Erfinder: CANNAS, Rita, CH-8600 Dübendorf (CH); BURCKHARDT, Urs, CH-8049 Zürich (CH)
(74) Vertreter: Sika Patent Attorneys
(86) Internationale Anmeldenummer: PCT/EP2012/075218
(87) Internationale Veröffentlichungsnummer: WO 2013/087689

(56) Entgegenhaltungen:
- WO-A1-2011/117225
- US-A- 4 871 854
- SYAMAL A: "Ferric benzoylacetanilides", CANADIAN JOURNAL OF CHEMISTRY, NRC RESEARCH PRESS, CA, Bd. 47, Nr. 10, 1. Januar 1969 (1969-01-01), Seiten 1693-1696, XP002600690, ISSN: 0008-4042, DOI: 10.1139/V69-278

## Beschreibung

### Technisches Gebiet

Die vorliegende Erfindung betrifft das Gebiet der Polyurethan-Zusammensetzungen sowie die Verwendung von speziellen Eisen (III) Komplexverbindungen als Katalysatoren für Polyurethan-Zusammensetzungen.

### Stand der Technik

Polyurethan-Zusammensetzungen sind seit langem bekannt und werden in vielen Bereichen eingesetzt. Klassisch wird in der Fachwelt zwischen einkomponentigen und zweikomponentigen Polyurethan-Zusammensetzungen unterschieden. Einkomponentige Polyurethan-Zusammensetzungen härten unter dem Einfluss von Luftfeuchtigkeit aus. Zweikomponentige Polyurethan-Zusammensetzungen enthalten als zweite Komponente eine Härterkomponente, welche im Wesentlichen Polyamine und/oder Polyole enthält. In beiden Fällen werden Isocyanatgruppen-haltige Verbindungen oder Prepolymere eingesetzt.

Um die Aushärtung zu beschleunigen, werden Katalysatoren beigefügt. Zwar ist eine Vielzahl von Polyurethan-Katalysatoren bekannt, allerdings ist eine Großzahl hinsichtlich der Urethanisierungsreaktion, d.h. der Reaktion von alkoholischen OH-Gruppen mit Isocyanatgruppen, nicht sonderlich selektiv, sondern katalysiert mehr oder weniger auch andere Reaktionen der Isocyanatgruppe, wie Allophanat- und Biuret-Bildung oder Cyclotrimerisierung. Insbesondere steht die Urethanisierungsreaktion meist in Konkurrenz zur Reaktion der Isocyanatgruppen mit Wasser, welche unter Freisetzung von gasförmigem Kohlendioxid zu Harnstoffgruppen führt. Diese Nebenreaktion ist bei vielen Polyurethan-Zusammensetzungen, insbesondere bei deren Anwendung als Kleb- und Dichtstoff, als Beschichtung oder Gießharz, störend, da sie bei der Aushärtung zu Blasenbildung und damit schlechter Formstabilität, geringerer Haftung, tieferer mechanischer Festigkeit, unbefriedigender Ästhetik sowie zu wenig reproduzierbaren Ergebnissen führt. Das für die Blasenbildung verantwortliche Wasser stammt entweder aus dem Restwasser-Gehalt der Bestandteile der Zusammensetzung, insbesondere der Polyole und der Füllstoffe, welche auch nach Trocknungsprozessen mehr oder weniger feucht sind und einen typischen Restwasser-Gehalt von 0,01 bis 0,5 Gew.-% aufweisen, oder aus der Umgebungsfeuchtigkeit, welche durch Diffusion aus der Luft oder aus den Substraten in die Zusammensetzung eindringt, was besonders bei hoher Luftfeuchtigkeit, porösen Substraten und/oder hydrophilen Polyolen, wie den in der Praxis oft eingesetzten Polyetherpolyolen, auftritt. Gerade die in der Praxis vielfach verwendeten Aminkatalysatoren, beispielsweise tertiäre Amine, und Zinnkatalysatoren, beispielsweise Dialkylzinncarboxylate, führen oft zu ausgeprägter Blasenbildung. Der Restwasser-Gehalt in der Polyurethan-Zusammensetzung bewirkt außerdem, dass hydrolyseempfindliche Katalysatoren, wie beispielsweise Bismutcarboxylate, bei längerem Aufbewahren der Zusammensetzung vor dem Gebrauch (Lagerung) deaktiviert werden, was sich negativ auf die Aushärtungsgeschwindigkeit und die mechanischen Eigenschaften auswirkt. Bei einigen bekannten Katalysatoren, beispielsweise den Dialkylzinncarboxylaten, ist darüber hinaus die Beständigkeit der ausgehärteten Zusammensetzung unter thermischer Belastung ungenügend, wobei der Katalysator einen Molekulargewichts-Abbau, d.h. eine Depolymerisation, unter Verlust an mechanischer Festigkeit verursacht. Weiterhin sind viele der bekannten Katalysatoren bei Raumtemperatur fest und in den Polyurethan-Ausgangsmaterialien oder den Weichmachern wenig löslich, so dass für ihren Einsatz in Zusammensetzungen, die bei Raumtemperatur härten, organische Lösungsmittel eingesetzt werden müssen. Schließlich sind manche der bekannten Katalysatoren, insbesondere solche auf Basis von Schwermetallverbindungen, toxikologisch bedenklich.

Die Verwendung von Eisen(III)-Komplexverbindungen als Katalysatoren für härtbare Massen, beispielsweise Polyurethan-Zusammensetzungen, ist bekannt. So beschreiben beispielsweise die US-A 4,871,854 und die US 2007/00110644 Eisen(III)-tris(acetylacetonat) als Katalysator. Dieser ist aber kristallin, hochschmelzend und in der Zusammensetzung nur in geringen Mengen löslich, so dass organische Lösungsmittel eingesetzt werden müssen oder bei hohen Aushärtungstemperaturen gearbeitet werden muss, um das katalytische Potential zu nutzen. Zwar sind die ebenfalls als Katalysatoren bekannten Eisen(III)-carboxylate, wie Eisen(III)-tris(2-ethylhexanoat), besser löslich, dafür sind sie aber hydrolyseempfindlich und werden deshalb beim Lagern relativ schnell deaktiviert.

### Darstellung der Erfindung

Aufgabe der vorliegenden Erfindung ist es, die vorstehend beschriebenen Nachteile des Standes der Technik zu beseitigen. Insbesondere besteht die Aufgabe der vorliegenden Erfindung darin, einen Katalysator bereitzustellen, der zu einer Verbesserung nachfolgender Eigenschaften bzw. zu einem ausgewogenen Verhältnis dieser führt.

Der Katalysator soll sich durch eine hohe katalytische Aktivität und Selektivität hinsichtlich der Urethanisierungsreaktion, d.h. der Reaktion von alkoholischen OH-Gruppen mit Isocyanatgruppen, auszeichnen und so einen raschen und durch Feuchtigkeit möglichst wenig gestörten Aufbau eines mechanisch hochwertigen Poly-urethan-Polymers aus polyfunktionellen Alkoholen (Polyolen) und Polyisocyanaten ermöglichen. Zum anderen soll der Katalysator eine ausreichende Hydrolyseresistenz besitzen, um unter üblichen Lagerbedingungen, d.h. bei Raumtemperatur oder bei leicht erhöhten Temperaturen, über mehrere Monate in einer restwasserhaltigen Polyol-Zusammensetzung ohne starken Aktivitätsverlust erhalten zu bleiben. Weiterhin soll der Katalysator die thermische Beständigkeit des ausgehärteten Polyurethan-Polymers möglichst wenig herabsetzen. Überdies soll der Katalysator bei Raumtemperatur oder bei leicht erhöhten Temperaturen flüssig sein bzw. in den Polyurethan-Ausgangsmaterialien oder in Weichmachern gut löslich sein, damit er in bei Raumtemperatur härtenden, lösungsmittelfreien Systemen auf einfache Weise eingesetzt werden kann. Letztendlich soll der Katalysator eine möglichst geringe Giftigkeit aufweisen.

Überraschenderweise wurde eine Eisen(III)-Komplexverbindung gemäß Anspruch 1 mit den gewünschten Eigenschaften gefunden. Diese aus der WO2011/117225 A1 bekannte Eisen(III)-Komplexverbindung besitzt die Formel Fe(L)ₓ(Y)₃₋ₓ, wobei x für 1, 2 oder 3 steht, Y für einen einfach negativ geladenen Liganden steht und L für einen Liganden der Formel (I) steht, 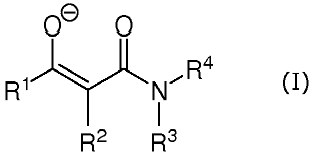 wobei R¹ und R² unabhängig voneinander für einen Wasserstoff-Rest, für einen einwertigen gesättigten oder ungesättigten Kohlenwasserstoff-Rest mit 1 bis 10 Kohlenstoff-Atomen, oder zusammen für einen zweiwertigen Alkylen-Rest mit 3 bis 6 Kohlenstoffatomen stehen und

R³ und R⁴ unabhängig voneinander für einen Wasserstoff-Rest, einen einwertigen gesättigten Kohlenwasserstoff-Rest, der gegebenenfalls Heteroatome enthält, mit 1 bis 12 Kohlenstoffatomen, oder zusammen für einen zweiwertigen Alkylen-Rest, der gegebenenfalls Heteroatome enthält, mit 3 bis 6 Kohlenstoffatomen stehen.

Der Ligand L der Formel (I) weist formal eine über die 1,3-Ketoamid-Struktur delokalisierte einfach negative Ladung auf. Er kann daher in verschiedenen Grenzstrukturen, beispielsweise in den nachfolgend dargestellten Grenzstrukturen, gezeichnet werden. Alle möglichen Grenzstrukturen des Liganden L der Formel (I) werden im Rahmen der vorliegenden Erfindung als gleichwertig angesehen.

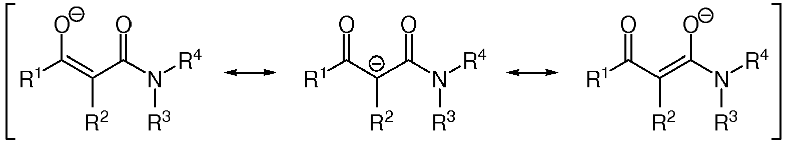

Der Ligand Y stellt einen beliebigen einfach negativ geladenen Liganden dar, insbesondere ein geeignetes organisches Anion, bevorzugt ein Carbonylat, besonders bevorzugt ein 1,3-Dicarbonylat, beispielsweise Acetylacetonat oder 2,2,6,6-Tetramethylheptan-3,5-dionat.

Die Eisen(III)-Komplexverbindung der Formel Fe(L)ₓ(Y)₃₋ₓ mit Eisen als Zentralatom und koordinativ an das Eisen gebundenen Liganden L und gegebenenfalls Y ist neutral und enthält mindestens einen Liganden L der Formel (I).

Weist die Eisen(III)-Komplexverbindung zwei oder drei Liganden L auf, d.h. x=2 oder 3 in obiger Formel, dann können die Liganden L gleich oder verschieden sein.

Vorzugsweise steht x in der Eisen(III)-Komplexverbindung der Formel Fe(L)ₓ(Y)₃₋ₓ für 3, da diese Komplexverbindungen besonders stabil sind. Die drei Liganden L der Formel (I) können gleich oder verschieden sein. Besonders bevorzugt liegen dabei drei gleiche Liganden L der Formel (I) vor.

In der Formel (I) stehen R¹ und R² unabhängig voneinander für einen Wasserstoff-Rest, für einen einwertigen gesättigten oder ungesättigten Kohlenwasserstoff-Rest mit 1 bis 10 Kohlenstoff-Atomen, oder zusammen für einen zweiwertigen Alkylen-Rest mit 3 bis 6 Kohlenstoffatomen.

Bei dem einwertigen gesättigten Kohlenwasserstoff-Rest mit 1 bis 10 Kohlenstoff-Atomen handelt es sich bevorzugt um einen Alkyl-Rest mit 1 bis 4 Kohlenstoffatomen, insbesondere einen Methyl- oder einen Butyl-Rest. Diese haben den Vorteil, dass die Komplexverbindung damit tendenziell flüssig oder gut löslich ist. Bei dem einwertigen ungesättigten Kohlenwasserstoff-Rest handelt es sich bevorzugt auch um einen Arylrest, insbesondere um einen Phenyl-Rest.
Besonders bevorzugt ist R² ein Wasserstoff-Rest, da die Komplexverbindung damit tendenziell besonders stabil ist.
Unter einem zweiwertigen Alkylen-Rest mit 3 bis 6 Kohlenstoffatomen wird ein Rest der Formel -(CH₂)ₙ- verstanden, wobei n 3 bis 6 bedeutet.
Bevorzugt bilden R¹ und R² zusammen einen zweiwertigen Alkylen-Rest mit 3 bis 4 Kohlenstoffatomen, insbesondere mit 3 Kohlenstoffatomen.
R³ und R⁴ stehen unabhängig voneinander für einen Wasserstoff-, einen einwertigen gesättigten Kohlenwasserstoff-Rest, der gegebenenfalls Heteroatome enthält, mit 1 bis 12 Kohlenstoffatomen, oder zusammen für einen zweiwertigen Alkylen-Rest, der gegebenenfalls Heteroatome enthält, mit 3 bis 6 Kohlenstoffatomen.
Bei dem einwertigen gesättigten Kohlenwasserstoff-Rest mit 1 bis 12 Kohlenstoffatomen handelt es sich bevorzugt um einen Alkylrest mit 1 bis 8 Kohlenstoffatomen, besonders bevorzugt um einen Methyl-, einen Ethyl-, einen Propyl-, einen Isopropyl-, einen Butyl-, einen Isobutyl-, einen Hexyl-, einen 2-Methyl-pentyl-, einen Octyl-, oder einen 2-Ethyl-hexyl-Rest. Dies hat den Vorteil, dass die Komplexverbindung damit tendenziell flüssig oder gut löslich ist. Vorzugsweise kann der einwertige gesättigte Kohlenwasserstoff-Rest mit 1 bis 12 Kohlenstoffatomen auch ein Cycloalkyl-Rest mit 5 bis 6 Kohlenstoffatomen, besonders bevorzugt 6 Kohlenstoffatomen, sein. Bei dem einwertigen gesättigten Kohlenwasserstoff-Rest mit Heteroatomen handelt es sich vorzugsweise um einen Hydroxyalkyl-Rest mit 1 bis 4 Kohlenstoffatomen, besonders bevorzugt um einen 2-Hydroxyethyl- oder 2-Hydroxypropyl-Rest. Dies hat den Vorteil, dass die Komplexverbindung damit tendenziell flüssig oder gut löslich ist und der Ligand bei der Aushärtung in das Polymer kovalent eingebunden werden kann. Bevorzugt ist auch ein Alkylether-Rest mit 1 bis 4 Kohlenstoffatomen, besonders bevorzugt ein 2-Methoxyethyl- oder ein 2-(2-Methoxy)ethoxyethyl-Rest, da die Komplexverbindung damit tendenziell flüssig oder gut löslich ist.

R³ kann zusammen mit R⁴ vorzugsweise auch einen zweiwertigen Alkylen-Rest der Formel -(CH₂)ₙ-X-(CH₂)ₙ- mit X=O, NR, wobei R ein einwertiger Alkyl-Rest mit 1 bis 4 Kohlenstoffatomen ist, oder S und n = 2 bis 4 bilden. Besonders bevorzugt ist n=2 und X=O oder NR.

Die Auswahl der bevorzugten Reste in den Liganden L der Formel (I) beruht vorzugsweise darauf, dass die entsprechenden 1,3-Ketoamide, welche als Ausgangsstoffe zur Herstellung der erfindungsgemäßen Eisen(III)-Komplexverbindung der Formel Fe(L)ₓ(Y)₃₋ₓ eingesetzt werden, einfach herstellbar und/oder kommerziell erhältlich und damit preisgünstig sind.

Bevorzugt sind Eisen(III)-Komplexverbindungen der Formel Fe(L)₃ mit drei gleichen Liganden L der Formel (I), wobei R¹ bis R⁴ die in der Tabelle genannten Bedeutungen besitzen.

| | **R¹** | **R²** | **R³** | **R⁴** |
|---|---|---|---|---|
| (1) | Alkyl-Rest mit 1-4 Kohlenstoffatomen | Wasserstoff-Rest | Alkyl-Rest mit 1-8 Kohlenstoffatomen | Alkyl-Rest mit 1-8-Kohlenstoffatomen |
| (2) | Phenyl-Rest | Wasserstoff-Rest | Alkyl-Rest mit 1-8 Kohlenstoffatomen | Alkyl-Rest mit 1-8-Kohlenstoffatomen |
| (3) | Alkyl-Rest mit 1-4 Kohlenstoffatomen | Wasserstoff-Rest | Alkylether-Rest mit 1-4 Kohlenstoffatomen | Alkylether-Rest mit 1-4 Kohlenstoffatomen |
| (4) | Alkylen-Rest mit 3-6 Kohlenstoffatomen | | Alkyl-Rest mit 1-8 Kohlenstoffatomen | |
| (5) | Alkyl-Rest mit 1-4 Kohlenstoffatomen | Wasserstoff-Rest | Alkylen-Rest der Formel -(CH₂)ₙ-X-(CH₂)ₙ- mit X=O oder NR und n=2 | |
| (6) | Alkyl-Rest mit 1-4 Kohlenstoffatomen | Wasserstoff-Rest | Cycloalkyl-Rest mit 5-6 Kohlenstoffatomen | Alkyl-Rest mit 1-8 Kohlenstoffatomen |
| (7) | Alkyl-Rest mit 1-4 Kohlenstoffatomen | Wasserstoff-Rest | Alkyl-Rest mit 1-8 Kohlenstoffatomen | Cycloalkyl-Rest mit 5-6 Kohlenstoffatomen |
| (8) | Phenyl-Rest | Wasserstoff-Rest | Alkylen-Rest der Formel (-CH₂)ₙ-X-(CH₂)ₙ- mit X=O oder NR und n=2 | |

Besonders bevorzugt sind hierbei die Fe(III)-Komplexverbindungen (1), (2) und (3), ganz besonders bevorzugt die Fe(III)-Komplexverbindung (1).

Ganz besonders bevorzugt sind Eisen(III)-Komplexverbindungen der Formel Fe(L)₃ mit drei gleichen Liganden L der Formel (I), wobei R¹ für einen Methyl-Rest, R² für einen Wasserstoff-Rest und R³ und R⁴ jeweils für einen EthylRest, oder R¹ für einen Methyl-Rest, R² für einen Wasserstoff-Rest und R³ und R⁴ für einen Alkylether-Rest mit 3 Kohlenstoffatomen, oder R¹ für einen Phenyl-Rest, R² für einen Wasserstoff-Rest und R³ und R⁴ für einen Butyl-Rest, oder R¹ für einen Butyl-Rest, R² für einen Wasserstoff-Rest und R³ und R⁴ für einen Butyl-Rest stehen.

Die Herstellung der Eisen(III)-Komplexverbindung der Formel Fe(L)₃(Y)₃₋ₓ erfolgt durch Umsetzung eines 1,3-Ketoamids der Formel 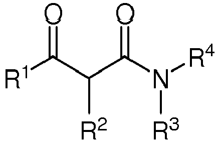 mit R¹, R², R³ und R⁴, wie vorstehend definiert, mit einem Eisen(III)-Salz oder Eisen(III)-Komplex. Bevorzugt ist der Einsatz von Eisen(III)-tris(acetylacetonat) und Eisen(III)-tris-(2-ethylhexanoat).

Das 1,3-Ketoamid kann dabei stöchiometrisch oder überstöchiometrisch eingesetzt werden. Bei einem überstöchiometrischen Einsatz des 1,3 Ketoamids weist die erfindungsgemäße Eisen(III)-Komplexverbindung tendenziell eine erhöhte Hydrolysestabilität und eine niedrigere Viskosität auf. Bevorzugt liegt das stöchiometrische Verhältnis zwischen dem Eisen (III)-Salz oder dem Eisen(III)-Komplex und dem 1,3-Ketoamid im Bereich von 1:3 bis 1:6.

Das vorzugsweise getrocknete Eisen(III)-Salz oder der Eisen(III)-Komplex wird mit dem 1,3-Ketoamid vermischt und die Mischung vorzugsweise unter Rühren während 1 bis 24 Stunden, bevorzugt etwa 4 Stunden, auf eine Temperatur von 50 bis 130 °C, insbesondere von etwa 90°C erwärmt. Anschlieβend wird das Reaktionsgemisch vorzugsweise im Vakuum von flüchtigen Bestandteilen befreit.

Das Eisen(III)-Salz oder der Eisen(III)-Komplex kann auch als Lösung, vorzugsweise in Ethylhexansäure, mit einem 1,3-Ketoamid versetzt und vorzugsweise unter Rühren während 1 bis 24 Stunden, bevorzugt etwa 3 Stunden, auf 50 bis 130°C, vorzugsweise etwa 80°C, erwärmt werden. Das Reaktionsgemisch wird dann vorzugsweise auf Raumtemperatur abgekühlt.

Die Eisen(III)-Komplexverbindungen können als Katalysator für härtbare Massen, bevorzugt für Polyurethan-Zusammensetzungen, verwendet werden. Die Eisen(III)-Komplexverbindung beschleunigt die Aushärtung von härtbaren Massen, welche zu Vernetzungsreaktionen fähige Reaktivgruppen aufweisen. Bevorzugt beschleunigt die Eisen(III)-Komplexverbindung die Aushärtung von zweikomponentigen Polyurethan-Zusammensetzungen, welche mit sich selbst und gegebenenfalls unter dem Einfluss von Feuchtigkeit über blockierte oder insbesondere freie Isocyanatgruppen vernetzen. Dabei wird vor allem die Urethanisierungsreaktion, d.h. die Reaktion von Isocyanatgruppen mit alkoholischen OH-Gruppen, beschleunigt. Die zu vernetzenden Zusammensetzungen können auch weitere zu Vernetzungsreaktionen fähige Reaktivgruppen, wie insbesondere Alkoxysilangruppen, enthalten. Vorzugsweise handelt es sich dabei um Trialkoxysilan-Gruppen, wie sie beispielsweise in Silan-Haftmitteln enthalten sind.

Die Eisen(III)-Komplexverbindungen können vorteilhafterweise als Katalysator in einer zweikomponentigen Polyurethan-Zusammensetzung eingesetzt werden. Diese umfasst neben der erfindungsgemäßen Eisen(III)-Komplexverbindung ein Polyol als erste Komponente sowie ein Polyisocyanat als zweite Komponente.
Als "zweikomponentig" wird eine Zusammensetzung bezeichnet, bei welcher die Bestandteile derselben in zwei verschiedenen Komponenten vorliegen, welche in voneinander getrennten Gebinden gelagert werden, und welche jeweils für sich lagerstabil sind. Erst kurz vor oder während der Applikation der Zusammensetzung werden die beiden Komponenten miteinander vermischt, worauf die vermischte Zusammensetzung aushärtet, wobei die Aushärtung unter Umständen erst durch die Einwirkung von Feuchtigkeit und/oder erhöhter Temperatur abläuft oder vervollständigt wird.

Mit "Poly" beginnende Substanznamen, wie Polyol oder Polyisocyanat, bezeichnen Substanzen, die formal zwei oder mehr der in ihrem Namen vorkommenden funktionellen Gruppen pro Molekül enthalten.

Der Begriff "Polyisocyanat" umfasst Verbindungen mit zwei oder mehr Isocyanatgruppen, unabhängig davon, ob es sich dabei um monomere Diisocyanate, oligomere Polyisocyanate oder Isocyanatgruppen aufweisende Polymere handelt.

Als Polyisocyanat geeignet ist beispielsweise ein Polyisocyanat in Form eines monomeren Di- oder Triisocyanates oder eines Oligomeren eines monomeren Diisocyanates oder eines Derivates eines monomeren Diisocyanates.

Als monomere Di- oder Triisocyanate geeignet sind beispielsweise 1,4-Tetramethylendiisocyanat, 2-Methylpentamethylen-1,5-diisocyanat, 1,6-Hexamethylendiisocyanat (HDI), 2,2,4- und 2,4,4-Trimethyl-1,6-hexamethylendiisocyanat (TMDI), 1,10-Decamethylendiisocyanat, 1,12-Dodecamethylendiisocyanat, Lysin- und Lysinesterdiisocyanat, Cyclohexan-1,3-und -1,4-diisocyanat, 1-Methyl-2,4- und -2,6-diisocyanatocyclohexan und beliebige Gemische dieser Isomeren, 1-Isocyanato-3,3,5-trimethyl-5-isocyanatomethyl-cyclohexan (=Isophorondiisocyanat oder IPDI), Perhydro-2,4'- und -4,4'-diphenylmethandiisocyanat (HMDI), 1,4-Diisocyanato-2,2,6-trimethylcyclohexan (TMCDI), 1,3- und 1,4-Bis-(isocyanatomethyl)-cyclohexan, m- und p-Xylylendiisocyanat (m- und p-XDI), m- und p-Tetramethyl-1,3- und -1,4-xylylendiisocyanat (m- und p-TMXDI), Bis-(1-Isocyanato-1-methylethyl)-naphthalin, Dimer- und Trimerfettsäureisocyanate, wie 3,6-Bis-(9-isocyanatononyl)-4,5-di-(1-heptenyl)-cyclohexen (Dimeryldiisocyanat), a,a,a',a',a",a"-Hexamethyl-1,3,5-mesitylentriisocyanat, 2,4- und 2,6-Toluylendiisocyanat und beliebige Gemische dieser Isomeren (TDI), 4,4'-, 2,4'- und 2,2'-Diphenylmethandiisocyanat und beliebige Gemische dieser Isomeren (MDI), Gemische aus MDI und MDI-Homologen (polymeres MDI oder PMDI), 1,3- und 1,4-Phenylendiisocyanat, 2,3,5,6-Tetramethyl-1,4-diisocyanatobenzol, Naphthalin-1,5-diisocyanat (NDI), 3,3'-Dimethyl-4,4'-diisocyanatodiphenyl (TODI), Dianisidindiisocyanat (DADI), 1,3,5-Tris-(isocyanatomethyl)-benzol, Tris-(4-isocyanatophenyl)-methan und Tris-(4-isocyanatophenyl)-thiophosphat.

Bevorzugte Polyisocyanate sind handelsübliche Diisocyanate. Besonders bevorzugt sind HDI, IPDI, TDI und MDI sowie Oligomere von Diisocyanaten und Isocyanatgruppen aufweisende Polyurethanpolymere (NCO-Prepolymere).

Als Polyole können beispielsweise die folgenden handelsüblichen Polyole oder Mischungen davon eingesetzt werden:
- Polyoxyalkylenpolyole, auch Polyetherpolyole oder Oligoetherole genannt, welche Polymerisationsprodukte von Ethylenoxid, 1,2-Propylenoxid, 1,2-oder 2,3-Butylenoxid, Oxetan, Tetrahydrofuran oder Mischungen davon sind, eventuell polymerisiert mit Hilfe eines Startermoleküls mit zwei oder mehreren aktiven Wasserstoffatomen wie beispielsweise Wasser, Ammoniak oder Verbindungen mit mehreren OH- oder NH-Gruppen wie beispielsweise 1,2-Ethandiol, 1,2- und 1,3-Propandiol, Neopentylglykol, Diethylenglykol, Triethylenglykol, die isomeren Dipropylenglykole und Tripropylenglykole, die isomeren Butandiole, Pentandiole, Hexandiole, Heptandiole, Octandiole, Nonandiole, Decandiole, Undecandiole, 1,3- und 1,4-Cyclohexandimethanol, Bisphenol A, hydriertes Bisphenol A, 1,1,1-Trimethylolethan, 1,1,1-Trimethylolpropan, Glycerin, Anilin, sowie Mischungen der vorgenannten Verbindungen. Eingesetzt werden können sowohl Polyoxyalkylenpolyole, die einen niedrigen Ungesättigtheitsgrad aufweisen (gemessen nach ASTM D-2849-69 und angegeben in Milliequivalent Ungesättigtheit pro Gramm Polyol (mEq/g)), hergestellt beispielsweise mit Hilfe von sogenannten Double Metal Cyanide Complex-Katalysatoren (DMC-Katalysatoren), als auch Polyoxyalkylenpolyole mit einem höheren Ungesättigtheitsgrad, hergestellt beispielsweise mit Hilfe von anionischen Katalysatoren wie NaOH, KOH, CsOH oder Alkalialkoholaten.
   Besonders geeignet sind Polyoxyalkylendiole oder Polyoxyalkylentriole, insbesondere Polyoxyethylen- und Polyoxypropylendi- und -triole. Speziell geeignet sind Polyoxyalkylendiole und -triole mit einem Ungesättigtheitsgrad tiefer als 0,02 mEq/g und mit einem Molekulargewicht im Bereich von 1.000 - 30.000 g/mol, sowie Polyoxypropylendiole und -triole mit einem Molekulargewicht von 400 - 8.000 g/mol.
   Ebenfalls besonders geeignet sind sogenannte Ethylenoxid-terminierte ("EO-endcapped", ethylene oxide-endcapped) Polyoxypropylenpolyole. Letztere sind spezielle Polyoxypropylenpolyoxyethylenpolyole, die beispielsweise dadurch erhalten werden, dass reine Polyoxypropylenpolyole, insbesondere Polyoxypropylendiole und -triole, nach Abschluss der Polypropoxylierungsreaktion mit Ethylenoxid weiter alkoxyliert werden und dadurch primäre Hydroxylgruppen aufweisen.
- Styrol-Acrylnitril- oder Acrylnitril-Methylmethacrylat-gepfropfte Polyetherpolyole.
- Polyesterpolyole, auch Oligoesterole genannt, hergestellt nach bekannten Verfahren, insbesondere der Polykondensation von Hydroxycarbonsäuren oder der Polykondensation von aliphatischen und/oder aromatischen Polycarbonsäuren mit zwei- oder mehrwertigen Alkoholen.
   Als Polyesterpolyole insbesondere geeignet sind solche, welche hergestellt sind aus zwei- bis dreiwertigen, insbesondere zweiwertigen, Alkoholen, wie beispielsweise Ethylenglykol, Diethylenglykol, Propylenglykol, Dipropylenglykol, Neopentylglykol, 1,4-Butandiol, 1,5-Pentandiol, 3-Methyl-1,5-hexandiol, 1,6-Hexandiol, 1,8-Octandiol, 1,10-Decandiol, 1,12-Dodecandiol, 1,12-Hydroxystearylalkohol, 1,4-Cyclohexandimethanol, Dimerfettsäurediol (Dimerdiol), Hydroxypivalinsäureneopentylglykolester, Glycerin, 1,1,1-Trimethylolpropan oder Mischungen der vorgenannten Alkohole, mit organischen Di- oder Tricarbonsäuren, insbesondere Dicarbonsäuren, oder deren Anhydride oder Ester, wie beispielsweise Bernsteinsäure, Glutarsäure, Adipinsäure, Trimethyladipinsäure, Korksäure, Azelainsäure, Sebacinsäure, Dodecandicarbonsäure, Maleinsäure, Fumarsäure, Dimerfettsäure, Phthalsäure, Phthalsäureanhydrid, Isophthalsäure, Terephthalsäure, Dimethylterephthalat, Hexahydrophthalsäure, Trimellithsäure und Trimellithsäureanhydrid, oder Mischungen der vorgenannten Säuren, sowie Polyesterpolyole aus Lactonen wie beispielsweise aus ε-Capro-lacton und Startern wie den vorgenannten zwei- oder dreiwertigen Alkoholen.
- Polycarbonatpolyole, wie sie durch Umsetzung beispielsweise der oben genannten - zum Aufbau der Polyesterpolyole eingesetzten - Alkohole mit Dialkylcarbonaten, Diarylcarbonaten oder Phosgen zugänglich sind.
- Mindestens zwei Hydroxylgruppen tragende Blockcopolymere, welche mindestens zwei verschiedene Blöcke mit Polyether-, Polyester- und/oder Polycarbonatstruktur der oben beschriebenen Art aufweisen, insbesondere Polyetherpolyesterpolyole.
- Polyacrylat- und Polymethacrylatpolyole.
- Polyhydroxyfunktionelle Fette und Öle, beispielsweise natürliche Fette und Öle, insbesondere Ricinusöl; oder durch chemische Modifizierung von natürlichen Fetten und Ölen gewonnene - sogenannte oleochemische - Polyole, beispielsweise die durch Epoxidierung ungesättigter Öle und anschließender Ringöffnung mit Carbonsäuren bzw. Alkoholen erhaltenen Epoxypolyester bzw. Epoxypolyether, oder durch Hydroformylierung und Hydrierung ungesättigter Öle erhaltene Polyole; oder aus natürlichen Fetten und Ölen durch Abbauprozesse wie Alkoholyse oder Ozonolyse und anschließender chemischer Verknüpfung, beispielsweise durch Umesterung oder Dimerisierung, der so gewonnenen Abbauprodukte oder Derivaten davon erhaltene Polyole. Geeignete Abbauprodukte von natürlichen Fetten und Ölen sind insbesondere Fettsäuren und Fettalkohole sowie Fettsäureester, insbesondere die Methylester (FAME), welche beispielsweise durch Hydroformylierung und Hydrierung zu Hydroxyfettsäureestern derivatisiert werden können.
- Polykohlenwasserstoffpolyole, auch Oligohydrocarbonole genannt, wie beispielsweise polyhydroxyfunktionelle Polyolefine, Polyisobutylene, Polyisoprene; polyhydroxyfunktionelle Ethylen-Propylen-, Ethylen-Butylen- oder Ethylen-Propylen-Dien-Copolymere; polyhydroxyfunktionelle Polymere von Dienen, insbesondere von 1,3-Butadien, welche insbesondere auch aus anionischer Polymerisation hergestellt sein können; polyhydroxyfunktionelle Copolymere aus Dienen wie 1,3-Butadien oder Diengemischen und Vinylmonomeren wie Styrol, Acrylonitril, Vinylchlorid, Vinylacetat, Vinylalkohol, Isobutylen und Isopren, beispielsweise polyhydroxyfunktionelle Acrylonitril/Butadien-Copolymere, wie sie beispielsweise aus Epoxiden oder Aminoalkoholen und carboxylterminierten Acrylonitril/Butadien-Copolymeren hergestellt werden können; sowie hydrierte polyhydroxyfunktionelle Polymere oder Copolymere von Dienen.

Die genannten Polyole weisen bevorzugt ein mittleres Molekulargewicht von 250 - 30.000 g/mol, insbesondere von 400 - 20.000 g/mol, auf und weisen ferner bevorzugt eine mittlere OH-Funktionalität im Bereich von 1,6 bis 3 auf.

Unter "Molekulargewicht" versteht man bei Oligomeren oder Polymeren stets das Molekulargewichtsmittel Mₙ.

Besonders bevorzugt ist der Einsatz von Polyetherpolyolen, vorzugsweise Polypropylenpolyolen und Polyethylen-polypropylen-Mischpolyolen, sowie Polyesterpolyolen und Polycarbonatpolyolen.

Die Eisen(III)-Komplexverbindung liegt vorzugsweise in der ersten Komponente vor, was den Vorteil hat, dass das auf katalytisch wirkende Verbindungen empfindliche Polyisocyanat in der zweiten Komponente in seiner Lagerstabilität (Haltbarkeit) nicht beeinträchtigt wird.

Die Eisen(III)-Komplexverbindung kann als alleiniger Katalysator oder auch zusammen mit anderen Katalysatoren, wie beispielsweise Bismut-, Zinn- oder Zirkoniumverbindungen oder tertiären Aminen, eingesetzt werden.

Die erfindungsgemäße zweikomponentige Polyurethan-Zusammensetzung kann gegebenenfalls weitere üblicherweise eingesetzte Hilfs- und Zusatzstoffe, beispielsweise Pigmente, Weichmacher bzw. Verdünner, Härter, Vernetzer, Kettenverlängerer, weitere Katalysatoren, Haftmittel, Stabilisatoren, Rheologiehilfsmittel und Trocknungsmittel, etc. enthalten.

Die Eisen(III)-Komplexverbindung, betrachtet als Menge elementares Eisen, liegt in der erfindungsgemäßen zweikomponentigen Polyurethan-Zusammensetzung bevorzugt in einer Menge von 0,001 bis 1 Gew.-%, besonders bevorzugt in einer Menge von 0,005 bis 0,5 Gew.-%, und ganz besonders bevorzugt in einer Menge von 0,01 bis 0,2 Gew.-%, bezogen auf das Gewicht der Zusammensetzung, vor. Zu hohe Mengen führen dazu, dass die Offenzeit bzw. Verarbeitungszeit der Zusammensetzung zu kurz ist, während der Einsatz zu geringer Mengen den Nachteil hat, dass die Zusammensetzung zu schwach katalysiert ist und damit zu langsam, unvollständig und/oder fehlerhaft aushärtet. In der erfindungsgemäßen zweikomponentigen Polyurethan-Zusammensetzung macht die erfindungsgemäße Eisen(III)-Komplexverbindung 0,01 bis 10, bevorzugt 0,05 bis 5, und besonders bevorzugt 0,1 bis 2 mmol-Equivalente Eisenatome auf 100 g der Zusammensetzung aus.

Wie bereits vorstehend erwähnt, ist die Eisen(III)-Komplexverbindung hinsichtlich der Urethanisierungsreaktion vergleichsweise aktiv sowie auch vergleichsweise selektiv. So zeichnet sich die Eisen(III)-Komplexverbindung gegenüber bekannten Eisen(III)-Komplexverbindungen durch eine deutlich höhere katalytische Aktivität aus. Die Aushärtung der erfindungsgemäßen zweikomponentigen Polyurethan-Zusammensetzung erfolgt im Allgemeinen rasch, insbesondere in deutlich kürzeren Aushärtungszeiten als bei Einsatz von bekannten Eisen(III)-Katalysatoren zur Aushärtung Die Selektivität der erfindungsgemäßen Eisen(III)-Komplexverbindung leidet jedoch nicht unter der erhöhten Aktivität; die Aushärtung erfolgt ohne Bildung von Blasen, selbst bei ungünstigen Bedingungen, wie hoher Temperatur, hoher Umgebungsfeuchte bzw. hohem Restwassergehalt der Zusammensetzung sowie bei Einsatz von Polyolen mit sekundären OH-Gruppen oder hydrophilen Polyolen. Die erfindungsgemäße Eisen(III)-Komplexverbindung ist thermisch und hydrolytisch vergleichsweise stabil, zersetzt sich selbst in einem restwasserhaltigen Polyol nur langsam und behält somit ihre katalytische Aktivität auch bei längerer Lagerdauer. Dennoch führt der Einsatz der erfindungsgemäßen Eisen(III)-Komplexverbindung zu guter Stabilität der ausgehärteten Polyurethan-Zusammensetzung unter thermischer Belastung. Die erfindungsgemäße Eisen(III)-Komplexverbindung ist ferner bei Raumtemperatur flüssig und/oder in Weichmachern oder Polyolen gut löslich und lässt sich somit in bei Raumtemperatur härtenden Systemen auf einfache Weise und insbesondere ohne Verwendung von flüchtigen organischen Lösungsmitteln (VOC) einsetzen. Schließlich weist die erfindungsgemäße Eisen(III)-Komplexverbindung eine relativ geringe Toxizität auf.

Die erfindungsgemäße zweikomponentige Polyurethan-Zusammensetzung kann in vielen Bereichen eingesetzt werden, beispielsweise als Vergussmasse, Dichtstoff, Klebstoff, Belag, Beschichtung, Lack, Voranstrich, Hartschaum, Weichschaum, Formteil, Elastomer, Faser, Folie oder Membran für Bau- und Industrieanwendungen, beispielsweise als Elektrovergussmasse, Spachtelmasse, Nahtabdichtung, Hohlraumversiegelung, Fugendichtstoff, Montageklebstoff, Karosserieklebstoff, Scheibenklebstoff, Sandwichelementklebstoff, Kaschierklebstoff, Laminatklebstoff, Verpackungsklebstoff, Holzklebstoff, Parkettklebstoff, Verankerungsklebstoff, Bodenbelag und -beschichtung, Balkon- und Dachbeschichtung, Betonschutzbeschichtung, Parkhausbeschichtung, Rohrbeschichtung, Korrosionsschutzbeschichtung, Textilbeschichtung, Holzlack, Dekorationslack, Primer, Möbelschaum, Polsterschaum, Filterschaum, Isolationsschaum, Schalldämmschaum, Abdichtungsschaum, Verpackungsschaum, Karosserieschaum, Modellbauplatte, Dämpfungselement, Dichtungselement, Reifen, Rollen, Lager, Walze, Förderband, Gummifaden, Schuhsohle, Gehäuse, Fensterprofil, Implantat, Schaumgummi, etc.

Bevorzugte Anwendungsgebiete sind Vergussmassen, Dichtstoffe, Klebstoffe, Beläge, Beschichtungen, Lacke, Voranstriche, Formteile und Elastomere für Bau- und Industrieanwendungen.

Außer in zweikomponentigen Polyurethan-Zusammensetzungen kann die erfindungsgemäße Eisen(III)-Komplexverbindung als Katalysator oder CoKatalysator auch in anderen härtbaren Massen eingesetzt werden, beispielsweise in einkomponentigen Polyurethan-Zusammensetzungen, in Epoxidharzen, Acrylaten und Silikonen.

### Beispiele

### Beschreibung der Messmethoden

Infrarotspektren wurden auf einem FT-IR Gerät 1600 von Perkin-Elmer gemessen (horizontale ATR-Messeinheit mit ZnSe-Kristall; Messfenster 4000-650 cm⁻¹). Flüssige Proben wurden unverdünnt als Filme aufgetragen, feste Proben wurden in CH₂Cl₂ gelöst. Die Absorptionsbanden sind in Wellenzahlen (cm⁻¹) angegeben.

¹H-NMR-Spektren wurden auf einem Spektrometer des Typs Bruker DPX-300 bei 300,13 MHz gemessen; die chemischen Verschiebungen δ sind angegeben in ppm relativ zu Tetramethylsilan (TMS). Echte und Pseudo-Kopplungsmuster wurden nicht unterschieden.

Die Viskosität wurde auf einem thermostatisierten Kegel-Platten-Viskosimeter Physica MCR 300 (Kegeldurchmesser 20 mm, Kegelwinkel 1°, Kegelspitze-Platten-Abstand 0,05 mm, Schergeschwindigkeit 0,1 bis 100 s⁻¹) gemessen.

UV-vis-Spektren von in Dichlormethan gelösten Proben (40 mg/l) in 1 cm Quarzküvetten wurden auf einem Spektrometer des Typs Varian Cary 50 im Wellenlängenbereich 800 - 200 nm gemessen. Angegeben sind die Extinktionsmaxima λₘₐₓ in nm und in Klammern die zugehörigen Extinktionskoeffizienten ε in I·g⁻¹ ·cm⁻¹.

### Herstellung der Eisen(III)-Komplexverbindungen

### Allgemeine Herstellvorschrift A

In einem Rundkolben wurden getrocknetes Eisen(III)-tris(acetylacetonat) und ein 1,3-Ketoamid (≥3 Equivalente/Fe) vermischt und die Mischung unter Rühren während 4 Stunden auf 90 °C erwärmt. Darauf wurde das Reaktionsgemisch im Vakuum von den flüchtigen Bestandteilen befreit.

### Allgemeine Herstellvorschrift B

In einem Rundkolben wurde eine Lösung von Eisen(III)-(2-ethylhexanoat) in 2-Ethylhexansäure (6% Fe) mit einem 1,3-Ketoamid (≥3 Equivalente/Fe) versetzt und unter Rühren während 3 Stunden auf 80 °C erwärmt. Darauf wurde das Reaktionsgemisch auf Raumtemperatur gekühlt.

### Beispiel 1: Eisen(III)-tris(N,N-diethyl-3-oxobutanamidat)

Gemäß der allgemeinen Herstellvorschrift A wurden 3,53 g Eisen(III)-tris(acetylacetonat) und 4,87 g N,N-Diethyl-3-oxobutanamid umgesetzt. Man erhielt 5,60 g eines roten hochviskosen Öls.
FT-IR: 2972, 2929, 1638, 1597, 1552, 1497, 1433, 1360, 1308, 1268, 1203, 1163, 1082, 1004, 961, 925, 827, 762, 725, 660.
UV-vis: 442 (0,4). (vgl. Eisen(III)-tris(acetylacetonat): 434 (0,7) und 354 (0,7).)

### Beispiel 2: Eisen(III)-tris(N,N-diethyl-3-oxobutanamidat)

Gemäß der allgemeinen Herstellvorschrift A wurden 4,40 g Eisen(III)-tris(acetylacetonat) und 9,29 g N,N-Diethyl-3-oxobutanamid umgesetzt. Man erhielt 10,70 g eines roten Öls.
FT-IR: 2970, 2932, 1722, 1637, 1595, 1557, 1512, 1492, 1452, 1432, 1374, 1356, 1309, 1272, 1203, 1163, 1081, 1004, 962, 827, 762, 659.

### Beispiel 3: Eisen(III)-tris(N,N-diethyl-3-oxobutanamidat)

Gemäß der allgemeinen Herstellvorschrift B wurden 1,72 g Eisen(III)-tris(2-ethylhexanoate) und 0,89 g N,N-Diethyl-3-oxobutanamid umgesetzt. Man erhielt 2,61 g eines roten Öls.
FT-IR:.2955, 2925, 2855, 1725, 1638, 1563, 1515, 1495, 1458, 1377, 1359, 1309, 1275, 1202, 1163, 1097, 1082, 1007, 963, 765, 663.

### Beispiel 4: Eisen(III)-tris(N,N-bis(2-methoxyethyl)-3-oxobutanamidat)

Gemäß der allgemeinen Herstellvorschrift A wurden 3,53 g Eisen(III)-tris(acetylacetonat) und 6,74 g N,N-Bis(2-methoxyethyl)-3-oxobutanamid umgesetzt. Man erhielt 7,69 g eines roten hochviskosen Öls.
FT-IR: 2980, 2925, 2888, 1639, 1556, 1510, 1452, 1357, 1273, 1192, 1002, 962, 927, 825, 763, 728, 700, 666.

### Beispiel 5: Eisen(III)-tris(N,N-dibutyl-3-oxo-3-phenylpropanamidat)

Gemäß der allgemeinen Herstellvorschrift A wurden 3,53 g Eisen(III)-tris(acetylacetonat) und 8,34 g N,N-Dibutyl-3-oxo-3-phenylpropanamid umgesetzt. Man erhielt 9,24 g eines roten hochviskosen Öls.
FT-IR: 2955, 2928, 2870, 1584, 1549, 1498, 1481, 1428, 1364, 1292, 1266, 1211, 1110, 1023, 917, 759, 732, 695.

### Beispiel 6: Eisen(III)-tris(N,N-dibutyl-3-oxo-butanamidat)

Gemäß der allgemeinen Herstellvorschrift A wurden 3,53 g Eisen(III)-tris(acetylacetonat) und 6,61 g N,N-Dibutyl-3-oxobutanamid umgesetzt. Man erhielt 7,50 g eines roten viskosen Öls.
FT-IR: 2955, 2928, 2870, 1598, 1557, 1511, 1461, 1429, 1366, 1291, 1267, 1228, 1199, 1155, 1111, 1007, 956, 762, 730, 697.

### Beispiel 7: Eisen(III)-tris(N,N-dibutyl-3-oxo-heptanamidat)

Gemäß der allgemeinen Herstellvorschrift A wurden 3,53 g Eisen(III)-tris(acetylacetonat) und 7,92 g N,N-Dibutyl-3-oxoheptanamid umgesetzt. Man erhielt 8,34 g eines roten viskosen Öls.
FT-IR: 2953, 2928, 2869, 1596, 1554, 1511, 1489, 1459, 1427, 1393, 1369, 1290, 1224, 1184, 1159, 1103, 1061, 959, 766, 730, 669.

### Zweikomponentige Polyurethan-Zusammensetzungen

### Beispiele 8 bis 9 und Vergleichsbeispiele V1 bis V5

Zur Herstellung der ersten Komponente wurden für jedes Beispiel ein Polyethertriol (Voranol^{®} CP 4755, von Dow) und ein Katalysator gemäß Tabelle 1 in einem Zentrifugalmischer (SpeedMixer™ DAC 150, FIackTek Inc.) während 30 sec. bei 3000 U/min. innig vermischt. Ein Teil der frisch hergestellten ersten Komponente wurde daraufhin in eine innenlackierte Aluminiumtube gefüllt, diese luftdicht verschlossen und während 7 Tagen in einem Umluftofen bei 60 °C gelagert.

Der verbleibende Teil der frisch hergestellten ersten Komponente wurde für jedes Beispiel auf die beschriebene Weise mit einem modifizierten, bei Raumtemperatur flüssigen Diphenylmethandiisocyanat (Desmodur^{®} CD-L, von Bayer) als zweite Komponente gemäß Tabelle 1 zu einer Polyurethan-Zusammensetzung vermischt.

Ebenso wurde für jedes Beispiel die während 7 Tagen bei 60 °C gelagerte erste Komponente mit der zweiten Komponente gemäß Tabelle 1 auf die gleiche Weise zu einer Polyurethan-Zusammensetzung vermischt.

| Beispiel | **8** | **9** | **V1** | **V2** | **V3** | **V4** | **V5** |
|---|---|---|---|---|---|---|---|
| *Erste Komponente:* | | | | | | | |
| Voranol^{®} CP 4755 | 50 | 50 | 50 | 50 | 50 | 50 | 50 |
| Katalysator **Beispiel 2** | 0,13 | - | - | - | - | - | - |
| Katalysator **Beispiel 7** | - | 0,09 | - | - | - | - | - |
| Fe(acac)₃^{a} | - | - | 0,21 | - | - | - | - |
| Eisenoctoat^{b} | - | - | - | 0,75 | - | - | - |
| DBTDL^{C} | - | - | - | - | 0,46 | - | - |
| Coscat^{®} 83^{d} | - | - | - | - | - | 0,02 | - |
| DABCO 33-LV^{® e} | - | - | - | - | - | - | 0,10 |
| mmol-Equiv./100g^{f} | 0,29 | 0,20 | 0,27 | 1,44 | 0,13 | 0,03 | 1,07 |

| *Zweite Komponente:* | | | | | | | |
|---|---|---|---|---|---|---|---|
| Desmodur^{®} CD-L | 5,10 | 5,10 | 5,10 | 5,10 | 5,10 | 5,10 | 5,10 |
| **Tabelle 1: Zweikomponentige Polyurethan-Zusammensetzungen** (Mengen in Gewichtsteilen). | | | | | | | |
| ^{a} 25%ige Suspension von Eisen(III)-tris(acetylacetonat) in N-Ethylpyrrolidon. | | | | | | | |
| ^{b} Eisen(III)-(2-ethylhexanoat) in 2-Ethylhexansäure (6% Fe). ^{c} 10%ige Lösung von Dibutylzinndilaurat in Diisodecylphthalat. ^{d} Bismut-tris(neodecanoat) in Neodecansäure (16% Bi, von Erbslöh). ^{e} 33%ige Lösung von 1,4-Diazabicyclo[2.2.2]octan in Dipropylenglykol (von Air Products). ^{f} mmol-Equivalente Metallatome bzw. Aminogruppen des Katalysators auf 100 g der Zusammensetzung. | | | | | | | |

Die Polyurethan-Zusammensetzungen wurden auf Aspekt, Zeit bis zur Klebefreiheit, Blasenbildung sowie Shore A-Härte geprüft, und zwar jeweils sowohl für die Zusammensetzung mit der frisch hergestellten ersten Komponente als auch für die Zusammensetzung mit der während 7 Tagen bei 60 °C gelagerten ersten Komponente. Ausschließlich für die Zusammensetzung mit der frisch hergestellten ersten Komponente wurde weiterhin die mechanischen Eigenschaften im Zugversuch gemessen, und zwar vor und nach verschiedenen Lagerungen zur beschleunigten Alterung der Proben.

Der Aspekt der Zusammensetzung wurde rein optisch beurteilt und als "klar", "trüb" oder inhomogen ("inh.") gewertet.

Zur Bestimmung der Zeit bis zur Klebefreiheit (Hautbildungszeit) wurden die raumtemperaturwarmen Zusammensetzungen in einer Schichtdicke von ca. 3 mm auf Pappkarton aufgetragen und im Normklima ("NK"; 23±1 °C, 50±5% relative Luftfeuchtigkeit) jeweils die Zeit bestimmt, die es dauerte, bis beim leichten Antippen der Oberfläche einer Zusammensetzung mittels einer Pipette aus LDPE erstmals keine Rückstände auf der Pipette mehr zurückblieben.

Die Blasenbildung wurde optisch beurteilt anhand der Menge ("viele", "einige", "keine") an Gasblasen, die in der für die Bestimmung der Hautbildungszeit angesetzten Zusammensetzung während deren Aushärtung auftrat.

Die Shore A-Härte wurde bestimmt nach DIN 53505 an während 7 Tagen im Normklima ausgehärteten Prüfkörpern.

Zur Bestimmung der mechanischen Eigenschaften im Zugversuch wurde von den Zusammensetzungen Filme von ca. 3 mm Dicke hergestellt, indem die Zusammensetzung in eine plane PTFE-Form gegossen und während 7 Tagen im Normklima ausgehärtet wurde. Es wurden klebfreie und elastische Filme erhalten. Aus den Filmen wurden Hanteln mit einer Länge von 75 mm, bei einer Steglänge von 30 mm und einer Stegbreite von 4 mm, ausgestanzt und ein Teil davon gemäß DIN EN 53504 bei einer Zuggeschwindigkeit von 200 mm/min auf Zugfestigkeit, Bruchdehnung und E-Modul (bei 0,5 bis 5,0% Dehnung) geprüft. Der verbleibende Teil der Hanteln wurde während 1 Tag bei 100 °C im Umluftofen, bzw. während 10 Tagen unter "Kataplasma" (40 °C und 100% relative Luftfeuchtigkeit), bzw. während 10 Tagen unter "Kataplasma" sowie 1 Tag bei 100 °C, gelagert, darauf jeweils während einem Tag im Normklima gehalten und gemäß DIN EN 53504 wie beschrieben geprüft.

Die Ergebnisse dieser Prüfungen sind in der Tabelle 2 aufgeführt.

| Beispiel | **8** | **9** | **V1** | **V2** | **V3** | **V4** | **V5** |
|---|---|---|---|---|---|---|---|
| *Zusammensetzung mit frisch hergestellter erster Komponente:* | | | | | | | |
| Aspekt | klar | klar | inh. | klar | klar | klar | klar |
| Hautbildungszeit (min.) | 32 | 20 | >180 | 40 | 10 | 3 | 15 |
| Shore A-Härte | 49 | 46 | 32 | 49 | 48 | 44 | 33 |
| Blasenbildung | keine | keine | keine | keine | einige | einige | einige |
| Zugfestigkeit (MPa): 7d/NK | 1,06 | 0,97 | 0,95 | 0,96 | 0,76 | 0,54 | 0,90 |
| +10d/Kataplasma | 0,89 | 0,92 | 0,94 | 0,85 | 0,71 | 0,79 | 0,82 |
| +1d/100°C | 0,91 | 0,86 | 0,84 | 0,76 | 0,60 | 0,73 | 0,86 |
| +10d/Kataplasma+1d/100°C | 0,88 | 0,89 | 0,88 | 0,85 | 0,65 | 0,73 | 0,89 |
| Bruchdehnung (%): 7d/NK | 82 | 69 | 80 | 107 | 65 | 42 | 100 |
| +10d/Kataplasma | 69 | 77 | 87 | 90 | 55 | 73 | 85 |
| +1d/100°C | 87 | 87 | 65 | 190 | 168 | 72 | 105 |
| +10d/Kataplasma+ld/100°C | 80 | 84 | 84 | 118 | 170 | 74 | 108 |
| E-Modul (MPa): 7d/NK | 2,33 | 2,24 | 2,06 | 1,48 | 1,68 | 1,46 | 1,44 |
| +10d/Kataplasma | 1,82 | 1,66 | 1,75 | 1,48 | 1,68 | 1,56 | 1,47 |
| +1d/100°C | 1,77 | 1,73 | 1,96 | 0,47 | 0,60 | 1,49 | 1,23 |
| +10d/Kataplasma+1d/100°C | 1,78 | 1,89 | 1,74 | 1,28 | 0,71 | 1,41 | 1,23 |

| *Zusammensetzung mit gelagerter erster Komponente:* | | | | | | | |
|---|---|---|---|---|---|---|---|
| Aspekt | klar | klar | inh. | klar | klar | klar | klar |
| Hautbildungszeit (min.) | 35 | 25 | 100 | 240 | 10 | 45 | 15 |
| Shore A-Härte | 49 | 48 | 37 | 48 | 48 | 45 | 32 |
| Blasenbildung | keine | keine | keine | keine | einige | einige | einige |
| **Tabelle 2: Eigenschaften der zweikomponentigen Polyurethan-Zusammensetzungen** | | | | | | | |

Aus der Tabelle 2 ist ersichtlich, dass die zweikomponentigen Polyurethanzusammensetzungen mit den erfindungsgemäßen Katalysatoren klare, homogene Mischungen darstellen, welche sowohl vor wie auch nach Lagerung vergleichsweise kurze Hautbildungszeiten aufweisen und blasenfrei zu einem Material mit vergleichsweise hoher Festigkeit und guter Beständigkeit aushärten.

### Beispiele 10 bis 11 und Vergleichsbeispiele V6 bis V10

Zur Herstellung der ersten Komponente wurden für jedes Beispiel ein Polyethertriol (Voranol^{®} CP 4755, von Dow), ein Polyetherdiol (Acclaim^{®} 4200, von Bayer) und ein Katalysator gemäß Tabelle 3 in einem Zentrifugalmischer (SpeedMixer™ DAC 150, FlackTek Inc.) während 30 sec. bei 3000 U/min. innig vermischt. Ein Teil der frisch hergestellten ersten Komponente wurde daraufhin in eine innenlackierte Aluminiumtube gefüllt, diese luftdicht verschlossen und während 7 Tagen in einem Umluftofen bei 60°C gelagert.

Der verbleibende Teil der frisch hergestellten ersten Komponente wurde für jedes Beispiel auf die beschriebene Weise mit einem modifizierten, bei Raumtemperatur flüssigen Diphenylmethandiisocyanat (Desmodur^{®} CD-L, von Bayer) als zweite Komponente gemäß Tabelle 3 zu einer Polyurethan-Zusammensetzung vermischt.

Ebenso wurde für jedes Beispiel die während 7 Tagen bei 60 °C gelagerte erste Komponente mit der zweiten Komponente gemäß Tabelle 3 auf die gleiche Weise zu einer Polyurethan-Zusammensetzung vermischt.

| Beispiel | **10** | **11** | **V6** | **V7** | **V8** | **V9** | **V10** |
|---|---|---|---|---|---|---|---|
| *Erste Komponente:* | | | | | | | |
| Voranol^{®} CP 4755 | 33,3 | 33,3 | 33,3 | 33,3 | 33,3 | 33,3 | 33,3 |
| Acclaim^{®} 4200 | 16,7 | 16,7 | 16,7 | 16,7 | 16,7 | 16,7 | 16,7 |
| Katalysator **Beispiel 2** | 0,15 | - | - | - | - | - | - |
| Katalysator **Beispiel 7** | - | 0,32 | - | - | - | - | - |
| Fe(acac)₃^{a} | - | - | 0,21 | - | - | - | - |
| Eisenoctoat^{b} | - | - | - | 0,90 | - | - | - |
| DBTDL^{c} | - | - | - | - | 0,49 | - | - |
| Coscat^{®} 83^{d} | - | - | - | - | - | 0,015 | - |
| DABCO 33-LV^{® e} | - | - | - | - | - | - | 0,14 |
| mmol-Equiv./100g^{f} | 0,34 | 0,70 | 0,27 | 1,74 | 0,14 | 0,02 | 1,50 |

| *Zweite Komponente:* | | | | | | | |
|---|---|---|---|---|---|---|---|
| Desmodur^{®} CD-L | 4,80 | 4,80 | 4,80 | 4,80 | 4,80 | 4,80 | 4,80 |
| **Tabelle 3: Zweikomponentige Polyurethan-Zusammensetzungen** (Mengen in Gewichtsteilen). | | | | | | | |
| ^{a} 25%ige Suspension von Eisen(III)-tris(acetylacetonat) in N-Ethylpyrrolidon. | | | | | | | |
| ^{b} Eisen(III)-(2-ethylhexanoat) in 2-Ethylhexansäure (6% Fe). ^{c} 10%ige Lösung von Dibutylzinn-dilaurat in Diisodecylphthalat. ^{d} Bismut-tris(neodecanoat) in Neodecansäure (16% Bi, von Erbslöh). ^{e} 33%ige Lösung von 1,4-Diazabicyclo[2.2.2]octan in Dipropylenglykol (von Air Products). ^{f} mmol-Equivalente Metallatome bzw. Aminogruppen des Katalysators auf 100 g der Zusammensetzung. | | | | | | | |

Die Polyurethan-Zusammensetzungen wurden wie für Beispiel 8 beschrieben auf Aspekt, Zeit bis zur Klebefreiheit, Blasenbildung sowie die mechanischen Eigenschaften im Zugversuch geprüft, und zwar jeweils nur für die Zusammensetzung mit der frisch hergestellten ersten Komponente.

Die Ergebnisse dieser Prüfungen sind in der Tabelle 4 aufgeführt.

| Beispiel | **10** | **11** | **V6** | **V7** | **V8** | **V9** | **V10** |
|---|---|---|---|---|---|---|---|
| *Zusammensetzung mit frisch hergestellter erster Komponente:* | | | | | | | |
| Aspekt | klar | klar | inh. | klar | klar | klar | klar |
| Hautbildungszeit (min.) | 65 | 27 | 120 | 75 | 27 | 40 | 35 |
| Blasenbildung | keine | keine | keine | keine | viele | keine | viele |
| Zugfestigkeit (MPa): 7d/NK | 0,96 | 0,98 | 0,82 | 0,45 | 0,77 | 0,98 | 0,65 |
| +10d/Kataplasma | 0,95 | 0,96 | 0,77 | 0,38 | 0,77 | 0,89 | 0,66 |
| +1d/100°C | 0,90 | 0,84 | 0,89 | 0,55 | 0,48 | 0,83 | 0,72 |
| +10d/Kataplasma+1d/100°C | 0,99 | 0,91 | 0,70 | 0,48 | 0,52 | 0,78 | 0,69 |
| Bruchdehnung (%): 7d/NK | 130 | 124 | 129 | 94 | 105 | 117 | 135 |
| +10d/Kataplasma | 125 | 118 | 111 | 92 | 105 | 94 | 148 |
| +1d/100°C | 159 | 192 | 124 | 341 | 341 | 108 | 193 |
| +10d/Kataplasma+1d/100°C | 161 | 125 | 103 | 152 | 303 | 96 | 181 |
| E-Modul (MPa): 7d/NK | 1,27 | 1,39 | 1,00 | 0,51 | 1,20 | 1,45 | 0,88 |
| +10d/Kataplasma | 1,41 | 1,67 | 1,06 | 0,60 | 1,30 | 1,56 | 0,81 |
| +1d/100°C | 1,09 | 0,75 | 1,33 | 0,14 | 0,20 | 1,34 | 0,69 |
| +10d/Kataplasma+1d/100°C | 1,12 | 1,31 | 1,05 | 0,32 | 0,28 | 1,33 | 0,65 |

| *Zusammensetzung mit gelagerter erster Komponente:* | | | | | | | |
|---|---|---|---|---|---|---|---|
| Aspekt | klar | klar | inh. | klar | klar | klar | klar |
| Hautbildungszeit (min.) | 73 | 32 | 105 | 300 | 30 | 210 | 35 |
| Blasenbildung | keine | keine | keine | einige | einige | einige | einige |
| **Tabelle 4: Eigenschaften der zweikomponentigen Polyurethan-Zusammensetzungen** | | | | | | | |

Aus der Tabelle 4 ist ersichtlich, dass die zweikomponentigen Polyurethanzusammensetzungen mit den erfindungsgemäßen Katalysatoren klare, homogene Mischungen darstellen, welche vergleichsweise kurze Hautbildungszeiten aufweisen und blasenfrei zu einem Material mit vergleichsweise hoher Festigkeit und guter Beständigkeit aushärten.

### Beispiele 12 bis 19

Wie für Beispiel 8 beschrieben wurden zur Herstellung der ersten Komponente jeweils ein Polyethertriol (Voranol^{®} CP 4755, von Dow) und ein Katalysator gemäß Tabelle 5 vermischt. Ein Teil der frisch hergestellten ersten Komponente wurde daraufhin in eine innenlackierte Aluminiumtube gefüllt, diese luftdicht verschlossen und während 7 Tagen in einem Umluftofen bei 60 °C gelagert.

Der verbleibende Teil der frisch hergestellten ersten Komponente wurde für jedes Beispiel auf die für Beispiel 8 beschriebene Weise mit einem modifizierten, bei Raumtemperatur flüssigen Diphenylmethandiisocyanat (Desmodur^{®} CD-L, von Bayer) als zweite Komponente gemäß Tabelle 5 zu einer Polyurethan-Zusammensetzung vermischt.

Ebenso wurde für jedes Beispiel die während 7 Tagen bei 60 °C gelagerte erste Komponente mit der zweiten Komponente gemäß Tabelle 5 auf die gleiche Weise zu einer Polyurethan-Zusammensetzung vermischt.

Die Polyurethan-Zusammensetzungen wurden wie für Beispiel 8 beschrieben auf Aspekt, Zeit bis zur Klebefreiheit, Blasenbildung, Shore A-Härte sowie die mechanischen Eigenschaften im Zugversuch geprüft.

Die Ergebnisse dieser Prüfungen sind in der Tabelle 6 aufgeführt.

| Beispiel | **12** | **13** | **14** | **15** | **16** | **17** | **18** | **19** |
|---|---|---|---|---|---|---|---|---|
| *Erste Komponente:* | | | | | | | | |
| Voranol^{®} CP 4755 | 30 | 30 | 30 | 30 | 30 | 30 | 30 | 30 |
| Katalysator **Beispiel 2** | 0,28 | 0,08 | - | - | - | - | - | - |
| Katalysator **Beispiel 1** | - | - | 0,19 | - | - | - | - | - |
| Katalysator **Beispiel 3** | - | - | - | 0,50 | - | - | - | - |
| Katalysator **Beispiel 4** | - | - | - | - | 0,27 | - | - | - |
| Katalysator **Beispiel 5** | - | - | - | - | - | 0,34 | - | - |
| Katalysator **Beispiel 6** | - | - | - | - | - | - | 0,25 | - |
| Katalysator **Beispiel 7** | - | - | - | - | - | - | - | 0,30 |
| mmol-Equiv./100g^{a} | 1,05 | 0,30 | 1,02 | 1,05 | 1,05 | 1,10 | 1,00 | 1,08 |

| *Zweite Komponente:* | | | | | | | | |
|---|---|---|---|---|---|---|---|---|
| Desmodur^{®} CD-L | 3,10 | 3,10 | 3,10 | 3,10 | 3,10 | 3,10 | 3,10 | 3,10 |
| **Tabelle 5: Zweikomponentige Polyurethan-Zusammensetzungen.** ^{a} mmol-Equivalente Eisenatome des Katalysators auf 100 g der Zusammensetzung. | | | | | | | | |

| Beispiel | **12** | **13** | **14** | **15** | **16** | **17** | **18** | **19** |
|---|---|---|---|---|---|---|---|---|
| *Zusammensetzung mit frisch hergestellter erster Komponente:* | | | | | | | | |
| Aspekt | klar | klar | klar | klar | klar | klar | klar | klar |
| Hautbildungszeit (min.) | 13 | 18 | 6 | 20 | 16 | 30 | 6 | 6 |
| Shore A-Härte | 49 | 50 | 46 | 46 | 45 | 43 | 49 | 46 |
| Blasenbildung | keine | keine | keine | keine | keine | keine | keine | keine |

| *Zusammensetzung mit gelagerter erster Komponente:* | | | | | | | | |
|---|---|---|---|---|---|---|---|---|
| Aspekt | klar | klar | klar | klar | klar | klar | klar | klar |
| Hautbildungszeit (min.) | 15 | 20 | 7 | 22 | 15 | 27 | 6 | 5 |
| Shore A-Härte | 48 | 49 | 46 | 48 | 49 | 48 | 47 | 48 |
| Blasenbildung | keine | keine | keine | keine | keine | keine | keine | keine |
| **Tabelle 6: Eigenschaften der zweikomponentigen Polyurethan-Zusammensetzungen.** | | | | | | | | |

Aus der Tabelle 6 ist ersichtlich, dass die zweikomponentigen Polyurethanzusammensetzungen mit den erfindungsgemäßen Katalysatoren klare, homogene Mischungen darstellen, welche sowohl vor wie auch nach Lagerung vergleichsweise kurze Hautbildungszeiten aufweisen und weitgehend blasenfrei zu einem Material mit guter Shore A-Härte aushärten.

## Patentansprüche

1. Verwendung einer Eisen(III)-Komplexverbindung der Formel Fe(L)ₓ(Y)₃₋ₓ, wobei x für 1, 2 oder 3 steht, Y für einen einfach negativ geladenen Liganden steht und L für einen Liganden der Formel (I) steht, 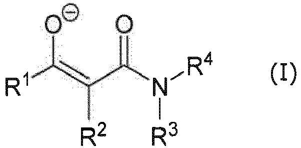
wobei R¹ und R² unabhängig voneinander für einen Wasserstoff-Rest, für einen einwertigen gesättigten oder ungesättigten Kohlenwasserstoff-Rest mit 1 bis 10 Kohlenstoff-Atomen, oder zusammen für einen zweiwertigen Alkylen-Rest mit 3 bis 6 Kohlenstoffatomen stehen und
R³ und R⁴ unabhängig voneinander für einen Wasserstoff-Rest, einen einwertigen gesättigten Kohlenwasserstoff-Rest, der gegebenenfalls Heteroatome enthält, mit 1 bis 12 Kohlenstoffatomen, oder zusammen für einen zweiwertigen Alkylen-Rest, der gegebenenfalls Heteroatome enthält, mit 3 bis 6 Kohlenstoffatomen stehen, als Katalysator für härtbare Massen, insbesondere für zweikomponentige Polyurethan-Zusammensetzungen.

2. Zweikomponentige Polyurethan-Zusammensetzungen, umfassend mindestens ein Polyol als erste Komponente, mindestens ein Polyisocyanat als zweite Komponente und mindestens eine Eisen(III)-Komplexverbindung der Formel Fe(L)ₓ(Y)₃₋ₓ, wobei x für 1, 2 oder 3 steht, Y für einen einfach negativ geladenen Liganden steht und L für einen Liganden der Formel (I) steht, 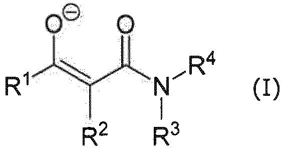
wobei R¹ und R² unabhängig voneinander für einen Wasserstoff-Rest, für einen einwertigen gesättigten oder ungesättigten Kohlenwasserstoff-Rest mit 1 bis 10 Kohlenstoff-Atomen, oder zusammen für einen zweiwertigen Alkylen-Rest mit 3 bis 6 Kohlenstoffatomen stehen und
R³ und R⁴ unabhängig voneinander für einen Wasserstoff-Rest, einen einwertigen gesättigten Kohlenwasserstoff-Rest, der gegebenenfalls Heteroatome enthält, mit 1 bis 12 Kohlenstoffatomen, oder zusammen für einen zweiwertigen Alkylen-Rest, der gegebenenfalls Heteroatome enthält, mit 3 bis 6 Kohlenstoffatomen stehen.

3. Zweikomponentige Polyurethan-Zusammensetzung nach Anspruch2, wobei das Polyol ein Polyetherpolyol und das Polyisocyanat ein Diisocyanat ist.

4. Zweikomponentige Polyurethan-Zusammensetzung nach mindestens einem der Ansprüche 2 bis 3, wobei die Eisen(III)-Komplexverbindung 0,01 bis 10, bevorzugt 0,05 bis 5, und besonders bevorzugt 0,1 bis 2 mmol-Equivalente Eisenatome auf 100 g der Zusammensetzung ausmacht.

5. Zweikomponentige Polyurethan-Zusammensetzung nach mindestens einem der Ansprüche 2 bis 4, wobei die Eisen(III)-Komplexverbindung in der ersten Komponente enthalten ist.

6. Verwendung der zweikomponentigen Polyurethan-Zusammensetzung nach mindestens einem der Ansprüche 2 bis 5 als Vergussmasse, Dichtstoff, Klebstoff, Belag, Beschichtung, Lack, Voranstrich, Formteil, Elastomer für Bau- und Industrieanwendungen.

## Claims

1. Use of an iron(III) complex of the formula Fe(L)ₓ(Y)₃₋ₓ, in which x stands for 1, 2 or 3, Y stands for a uninegatively charged ligand, and L stands for a ligand of formula (I), 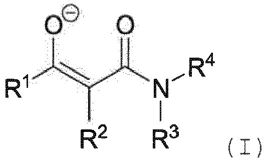 wherein R¹ and R² independently stand for a hydrogen group or for a monovalent saturated or unsaturated hydrocarbon group having 1 to 10 carbon atoms, or together stand for a divalent alkylene group having 3 to 6 carbon atoms, and R³ and R⁴ independently stand for a hydrogen group or for a monovalent saturated hydrocarbon group, optionally containing heteroatoms and having 1 to 12 carbon atoms, or together stand for a divalent alkylene group, optionally containing heteroatoms and having 3 to 6 carbon atoms, as a catalyst for curable substances, in particular for two-component polyurethane compositions.

2. Two-component polyurethane compositions, comprising at least one polyol as the first component, at least one polyisocyanate as the second component, and at least one iron(III) complex of the formula Fe(L)ₓ(Y)₃₋ₓ, in which x stands for 1, 2 or 3, Y stands for a uninegatively charged ligand, and L stands for a ligand of formula (I), 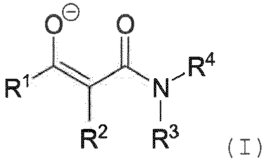 wherein R¹ and R² independently stand for a hydrogen group or for a monovalent saturated or unsaturated hydrocarbon group having 1 to 10 carbon atoms, or together stand for a divalent alkylene group having 3 to 6 carbon atoms, and R³ and R⁴ independently stand for a hydrogen group or for a monovalent saturated hydrocarbon group, optionally containing heteroatoms and having 1 to 12 carbon atoms, or together stand for a divalent alkylene group, optionally containing heteroatoms and having 3 to 6 carbon atoms.

3. The two-component polyurethane composition according to claim 2, wherein the polyol is a polyether polyol and the polyisocyanate is a diisocyanate.

4. The two-component polyurethane composition according to at least one of claims 2 and 3, wherein the iron(III) complex makes up 0.01 to 10, preferably 0.05 to 5, and particularly preferably 0.1 to 2 mmol-equivalent iron atoms to 100 g of the composition.

5. The two-component polyurethane composition according to at least one of claims 2 to 4, wherein the iron(III) complex is contained in the first component.

6. The use of the two-component polyurethane composition according to at least one of claims 2 to 5 as a casting compound, sealant, adhesive agent, covering, coating, varnish, undercoating, molded element or elastomer for constructional and industrial applications.

## Revendications

1. Utilisation d'un composé complexe de fer (III) de formule Fe(L)ₓ(Y)₃₋ₓ, dans laquelle x représente 1, 2 ou 3., Y représente un ligand à une charge négative et L représente un ligand de formule (I) 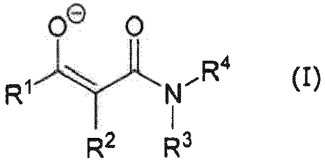
dans laquelle R¹ et R² représentent indépendamment l'un de l'autre un radical hydrogène, un radical hydrocarboné monovalent saturé ou insaturé de 1 à 10 atomes de carbone, ou ensemble un radical alkylène bivalent de 3 à 6 atomes de carbone, et
R³ et R⁴ représentent indépendamment l'un de l'autre un radical hydrogène, un radical hydrocarboné monovalent saturé, qui contient éventuellement des hétéroatomes, de 1 à 12 atomes de carbone, ou ensemble un radical alkylène bivalent, qui contient éventuellement des hétéroatomes, de 3 à 6 atomes de carbone, en tant que catalyseur pour matériaux durcissables, notamment pour compositions de polyuréthane bicomposantes.

2. Compositions de polyuréthane bicomposantes, comprenant au moins un polyol en tant que premier composant, au moins un polyisocyanate en tant que second composant et au moins un composé complexe de fer (III) de formule Fe(L)ₓ(Y)₃₋ₓ, dans laquelle x représente 1, 2 ou 3, Y représente un ligand à une charge négative et L représente un ligand de formule (I) 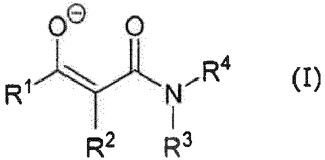
dans laquelle R¹ et R² représentent indépendamment l'un de l'autre un radical hydrogène, un radical hydrocarboné monovalent saturé ou insaturé de 1 à 10 atomes de carbone, ou ensemble un radical alkylène bivalent de 3 à 6 atomes de carbone, et
R³ et R⁴ représentent indépendamment l'un de l'autre un radical hydrogène, un radical hydrocarboné monovalent saturé, qui contient éventuellement des hétéroatomes, de 1 à 12 atomes de carbone, ou ensemble un radical alkylène bivalent, qui contient éventuellement des hétéroatomes, de 3 à 6 atomes de carbone.

3. Composition de polyuréthane bicomposante selon la revendication 2, dans laquelle le polyol est un polyéther-polyol et le polyisocyanate est un diisocyanate.

4. Composition de polyuréthane bicomposante selon au moins l'une quelconque des revendications 2 à 3, dans laquelle le composé complexe de fer (III) représente 0,01 à 10, de préférence 0,05 à 5, et de manière particulièrement préférée 0,1 à 2 équivalents mmol d'atomes de fer pour 100 g de la composition.

5. Composition de polyuréthane bicomposante selon au moins l'une quelconque des revendications 2 à 4, dans laquelle le composé complexe de fer (III) est contenu dans le premier composant.

6. Utilisation de la composition de polyuréthane bicomposante selon au moins l'une quelconque des revendications 2 à 5 en tant que matériau de scellement, matériau d'étanchéité, adhésif, garniture, revêtement, vernis, apprêt, pièce moulée, élastomère pour des applications dans le bâtiment et l'industrie.
